(19) Europäisches Patentamt
European Patent Office
Office européen des brevets

(11) Veröffentlichungsnummer: **0 165 537**
**B1**

(12) # EUROPÄISCHE PATENTSCHRIFT

(45) Veröffentlichungstag der Patentschrift: **17.01.90**

(21) Anmeldenummer: **85107108.4**

(22) Anmeldetag: **10.06.85**

(51) Int. Cl.[4]: **C 07 D 263/58**, C 07 D 271/06, C 07 D 277/68, C 07 D 277/34, C 07 D 285/12, C 07 D 285/08, C 07 D 413/12, C 07 D 417/12, C 07 D 277/56

(54) **Verfahren zur Herstellung von Heteroaryloxyacetamiden.**

(30) Priorität: **20.06.84 DE 3422861**

(43) Veröffentlichungstag der Anmeldung: **27.12.85 Patentblatt 85/52**

(45) Bekanntmachung des Hinweises auf die Patenterteilung: **17.01.90 Patentblatt 90/03**

(84) Benannte Vertragsstaaten: **BE CH DE FR GB IT LI NL**

(56) Entgegenhaltungen:
**EP-A-0 005 501**
**EP-A-0 018 497**
**EP-A-0 044 497**

**CHEMISCHE BERICHTE, Band 98, Nr. 2, Februar 1965, Seiten 487-503, Weinheim, DE; F. WEYGAND et al.: "Über einen ungewöhnlich leichten Austausch der Sulfonylgruppe gegen nucleophile Reste bei 2.2.2-Trifluor-1-äthansulfonyl-N-acyl-äthylaminen"**

**Die Akte enthält technische Angaben, die nach dem Eingang der Anmeldung eingereicht wurden und die nicht in dieser Patentschrift enthalten sind.**

(73) Patentinhaber: **BAYER AG**
**D-5090 Leverkusen 1 Bayerwerk (DE)**

(72) Erfinder: **Schmidt, Thomas, Dr.**
**Ginsterweg 9**
**D-5657 Haan 1 (DE)**
Erfinder: **Diehr, Hans-Joachim, Dr.**
**Höhe 35**
**D-5600 Wuppertal 11 (DE)**

**EP 0 165 537 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die Erfindung betrifft ein neues Verfahren zur Herstellung von bekannten, herbizid wirksamen Heteroaryloxyacetamiden.

Es ist bereits bekannt, daß man Heteroaryloxyacetamide, wie z. B. das herbizid wirksame N-Methyl-(5-trifluormethyl-1,3,4-thiadiazol-2-yl)-oxyacetanilid, erhält, wenn man Hydroxyacetamide, wie z. B. das N-Methylhydroxyacetanilid, mit entsprechenden halogensubstituierten Heteroaromaten, wie z. B. dem 2-Chlor-5-trifluormethyl-1,3,4-thiadiazol, in Gegenwart eines Säurebindemittels unter Verwendung eines organischen oder wäßrigen Verdünnungsmittels umsetzt (vgl. EP-A-0 005 501, EP-A-0 018 487, DE-OS3 0 38 636 und DE-OS3 148 839). Nachteilig bei diesem Verfahren ist die Verwendung von halogensubstituierten Heteroaromaten als Ausgangsstoffe, da sich diese häufig nicht in größeren Mengen auf einfachem Wege in guter Ausbeute und Reinheit darstellen lassen und, wenn man auf aufwendige Reinigungsoperationen verzichtet, die in den Ausgangsstoffen enthalten Nebenprodukte und Verunreinigungen auch in die Endprodukte mit eingeschleppt werden.

Es wurde nun gefunden, (daß man die bekannten, herbizid wirksamen Heteroaryloxyacetamide der allgemeinen Formel (I)

$$R\text{-}O\text{-}CH_2\text{-}CO\text{-}N\begin{matrix}R^1\\ |\\ \\ |\\ R^2\end{matrix} \qquad (I)$$

in welcher R für einen gegebenenfalls einfach oder mehrfach, gleich oder verschieden substituierten 5-gliedrigen aromatischen Heterocyclus steht, der auch benzoannelliert sein kann und der als Heteroatome 1 bis 3 Stickstoffatome und zusätzlich ein Sauerstoffatom oder ein Schwefelatom enthalten kann, wobei als Substituenten infrage kommen: Halogen, Cyano, Nitro, geradkettiges oder verzweigtes Alkyl, Alkoxy oder Alkylthio mit jeweils bis zu 4 Kohlenstoffatomen, geradkettiges oder verzweigtes Halogenalkyl mit bis zu 4 Kohlenstoffatomen und bis zu 9 gleichen oder verschiedenen Halogenatomen, Aralkyl, Aralkoxy oder Aralkylthio mit jeweils 6 oder 10 Kohlenstoffatomen im Arylteil und 1 bis 2 Kohlenstoffatomen im Alkylteil sowie gegebenenfalls ein- bis dreifach, gleich oder verschieden durch $C_{1-4}$-Alkyl, $C_{1-4}$-Alkoxy, Halogen oder $C_{1-4}$-Halogenalkyl substituiertes Aryl mit 6 oder 10 Kohlenstoffatomen, und

$R^1$ und $R^2$ unabhängig voneinander für Wasserstoff, geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatomen (wobei als Substituenten insbesondere Alkylreste mit 1 bis 4 Kohlenstoffatomen in Frage kommen), für geradkettiges oder verzweigtes Alkoxy und Alkoxyalkyl mit 1 bis 8 Kohlenstoffatomen, für Halogenalkyl mit 1 bis 8 Kohlenstoffatomen und 1 bis 5 Halogenatomen (insbesondere Fluor, Chlor und Brom), für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 oder 10 Kohlenstoffatomen stehen, wobei als Substituenten in Frage kommen:

Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, insbesondere Fluor, Chlor oder Brom, sowie Nitro, oder

$R^1$ und $R^2$ zusammen mit dem Stickstoffatom, an das sie gebunden sind, für einen gegebenenfalls einfach oder mehrfach, gleich oder verschieden substituierten gesättigten oder ungesättigten, 5- bis 7-gliedrigen Heterocyclus stehen, der bis zu 2 weitere Heteroatome, insbesondere Stickstoff oder Sauerstoff enthalten kann, wobei als Substituenten in Frage kommen: geradkettiges oder verzweigtes Alkyl mit 1 bis 6 Kohlenstoffatomen, auch in Form eines annellierten Ringsystems, Aryl mit 6 oder 10 Kohlenstoffatomen, auch in Form eines annellierten Ringsystems oder Dioxyalkylen mit 2 bis 3 Kohlenstoffatomen.

In guten Ausbeuten und hoher Reiheit erhält, wenn man sulfonylierte Heteroaromaten der allgemeinen Formel (II)

$$R\text{-}SO_2\text{-}R' \qquad (II)$$

in welcher

R die oben angegebene Bedeutung hat und

R' für niederes Alkyl oder für gegebenenfalls substituiertes Benzyl steht,

mit α- Oxyacetamiden der allgemeinen Formel (III),

$$R'' - O - CH_2 - CO - N\begin{matrix}R^1\\ |\\ \\ |\\ R^2\end{matrix} \qquad (III)$$

in welcher

R" für Wasserstoff oder Acyl steht und

$R^1$ und $R^2$ die oben angegebene Bedeutung haben,

in Gegenwart einer anorganischen Base als Säureakzeptor und eines organischen oder anorganischen Lösungsmittels als Verdünnungsmittel, gegebenenfalls in Gegenwart eines Phasentransferkatalysators, bei Temperaturen von -50°C bis +150°C umsetzt.

Es ist als ausgesprochen überraschend anzusehen, daß die Umsetzung von sulfonylierten Heteroaromaten der Formel (II) mit α-Oxyacetamiden der Formel (III) in Gegenwart von einfachen anorganischen Basen wie wäßriger Natronlauge oder Kaliumcarbonat in guten Ausbeuten gelingt, da aus dem Stand der Technik bekannt ist, daß derartige Substitutionsreaktionen nur unter Katalyse von stark basischen und hydrolyseempfindlichen organischen Basen wie beispielsweise Alkoholaten gelingen (vgl. E. Hoggarth, J. Chem. Soc. *1949*, 3311 - 3325). Außerdem ist es überraschend, daß unter den Reaktionsbedingungen in dem stark basischen Reaktionsmilieu die Amidfunktionen sowohl der Ausgangsprodukte der Formel (III) als auch der Endprodukte der Formel (I) nicht verseift werden, sondern unverändert erhalten bleiben.

Ein Vorteil des erfindungsgemäßen Verfahrens besteht darin, daß sich die als Ausgangsverbindungen verwendeten sulfonylierten Heteroaromaten leicht, in großen Mengen und hervorragender Reinheit aus den entsprechenden, ebenfalls gut zugänglichen Thioethern herstellen lassen, ohne daß aufwendige Reinigungsoperationen erforderlich werden. Ein weiterer Vorteil des neuen Verfahrens liegt in den hohen Ausbeuten an Heteroaryloxyacetamiden der Formel (I), die die Ausbeute der bekannten Verfahren übertreffen.

Das erfindungsgemäße Verfahren betrifft bevorzugt Verbindungen der Formel (I), bei denen

R für einen der Heterocyclen

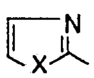, 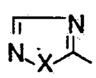, 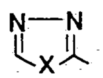 oder 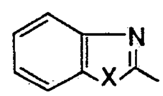

steht, wobei X jeweils für Sauerstoff oder Schwefel steht, die gegebenenfalls einfach oder mehrfach, gleich oder verschieden substituiert sein können, wobei als Substituenten insbesondere genannt seien: Fluor, Chlor, Brom, Cyano, Nitro, Methyl, Ethyl, n- und i-Propyl, n-, i-, s- und t-Butyl, Methoxy, Ethoxy, Methylthio, Trifluormethyl, Trichlormethyl, Fluordichlormethyl, Difluorchlormethyl, Benzyl, Benzyloxy, Benzylthio, sowie gegebenenfalls ein- bis dreifach, gleich oder verschieden durch Methyl, Methoxy, Chlor oder Trifluormethyl substituiertes Phenyl, und

$R^1$ und $R^2$ unabhängig voneinander für Wasserstoff, für geradkettiges oder verzweigtes Alkyl mit 1 bis 6 Kohlenstoffatomen, für geradkettiges oder verzweigtes Alkenyl und Alkinyl mit jeweils 2 bis 8 Kohlenstoffatomen, für gegebenenfalls ein- bis dreifach, gleich oder verschieden durch Methyl oder Ethyl substituiertes Cycloalkyl bzw. Cycloalkenyl mit 5 bis 7 Kohlenstoffatomen, für verzweigtes oder geradkettiges Alkoxy und Alkoxyalkyl mit 1 bis 6 Kohlenstoffatomen, für Halogenalkyl mit 1 bis 6 Kohlenstoffatomen und 1 bis 5 Halogenatomen (insbesondere Fluor, Brom und Chlor), für Benzyl sowie für gegebenenfalls ein- bis dreifach, gleich oder verschieden substituiertes Phenyl stehen, wobei als Substituenten besonders bevorzugt sind:

Methyl, Ethyl, Methoxy, Methylthio, Trifluormethyl, Trifluormethoxy, Trifluormethylthio, Fluor, Chlor, Nitro, oder

$R^1$ und $R^2$ zusammen mit dem Stickstoffatomen, an das sie gebunden sind, für die gegebenenfalls ein- bis dreifach, gleich oder verschieden substituierten Heterocyclen der Formeln:

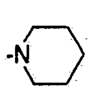 ; 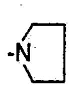 ; 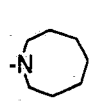 ; 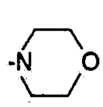 ; 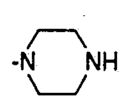 ; 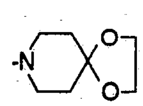 ;

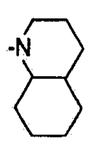 ; 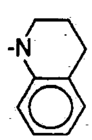 stehen,

wobei als Substituenten besonders bevorzugt sind: Methyl, Ethyl und Phenyl.

Verwendet man als Ausgangsstoffe beispielsweise N-Methylhydroxyacetanilid und 2-Methylsulfonyl-5-trifluormethyl-1,3,4-thiadiazol, so kann der Reaktionsablauf des erfindungsgemäßen Verfahrens durch das folgende Formelschema dargestellt werden:

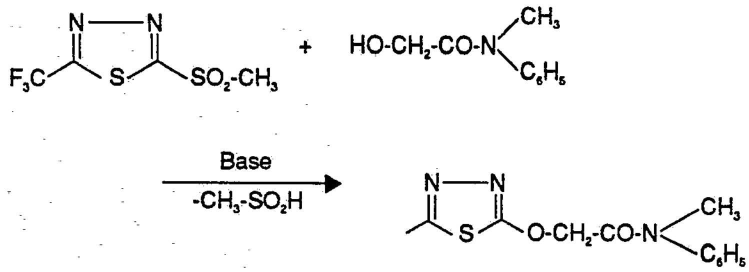


Die für das erfindungsgemäße Verfahren als Ausgangsstoffe benötigten sulfonylierten Heteroaromaten sind durch die Formel (II) allgemein definiert. In dieser Formel steht R vorzugsweise für diejenigen heterocyclischen Reste, die bei der Beschreibung der entsprechenden Reste der Verbindungen der Formel (I) als bevorzugt genannt wurden, R' steht vorzugsweise für Methyl oder Ethyl.

Die sulfonylierten Heteroaromaten der Formel (II) sind bekannt oder können nach bekannten Verfahren in einfacher Weise erhalten werden (vgl. z. B. J. Chem. Soc. *1949,* S. 1918 - 1923; J. Chem. Soc. *1949,* S. 3311 - 3315; ferner Chem. Abstr. Vol. *67*, 89998k (1967); Chem. Abstr. Vol *68*, 2169 q (1968); US-PS2 562 284; DE-0S2 533 604; DE-0S2 533 605; DE-0S3 027 483; DE-OS3 145 422; Eur. J. Med. Chem *1978* - 13 (Nr. 2), S. 171 - 175; J. Heterocyclic Chem. *13* (1976), S. 491 - 496).

Die für das erfindungsgemäße Verfahren weiterhin als Ausgangsstoffe benötigten α-Oxyacetamide sind durch die Formel (III) allgemein definiert. In dieser Formel stehen $R^1$ und $R^2$ vorzugsweise für diejenigen Reste, die bei der Beschreibung der entsprechenden Reste der Verbindungen der Formel (I) als bevorzugt genannt wurden, R" steht vorzugsweise für Wasserstoff oder für einen Formyl-, Acetyl- oder Benzoylrest.

Die Verbindungen der Formel (III) sind bekannt oder können nach an sich bekannten Verfahren auf einfache Weise hergestellt werden (vgl. z. B. EP-A-0 005 501, EP-A-0 014 409, EP-A-0 018 497, EP-A-0 029 171, DE-OS2 201 432, DE-0S2 904 490, DE-OS3 038 598).

Als Verdünnungsmittel für das erfindungsgemäße Verfahren kommen organische oder anorganische Lösungsmittel in Frage. Bevorzugt sind Kohlenwasserstoffe, wie Toluol oder Cyclohexan, Halogenkohlenwasserstoffe, wie Methylenchlorid, Chloroform, Dichlorethan oder Chlorbenzol, Ketone, wie Aceton oder Methylisobutylketon, Ether, wie Diethylether, Diisopropylether oder Methyl-t-butylether, Alkohole, wie Methanol, Ethanol oder Isopropanol, Amide, wie Dimethylformamid oder Dimethylacetamid, Sulfoxide, wie Dimethylsulfoxid, Wasser oder wäßrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt ist Natriumchlorid.

Das erfindungsgemäße Verfahren wird unter Verwendung eines Säureakzeptors durchgeführt. Als solche Säurebindemittel werden vorzugsweise stark basische Alkali- und Erdalkalimetallverbindungen, beispielsweise Oxide, wie z. B. Natrium-, Kalium-, Magnesium- und Calciumoxid; Hydroxide, wie z. B. Natrium-, Kalium-, Magnesium- und Calciumhydroxid und/oder Carbonate, wie z. B. Natrium-, Kalium-, Magnesium- und Calciumcarbonat verwendet.

Der Zusatz von 0,01 bis 10 Gew.- % (bezogen auf eingesetztes α-Oxyacetamid der Formel (III)) eines Phasentransferkatalysators mag sich in einigen Fällen als vorteilhaft erweisen. Als Beispiele für solche Katalysatoren seien genannt:

Tetrabutylammoniumiodid, Tetrabutylammoniumbromid, Tributyl-methylphosphoniumbromid, Trimethyl-$C_{13}/C_{15}$-alkyl-ammoniumchlorid, Dibenzyl-dimethyl-ammonium-methylsulfat, Dimethyl-$C_{12}/C_{14}$-alkyl-benzylammoniumchlorid, Tetrabutylammoniumhydroxid, 18-Krone-6, Triethylbenzylammoniumchlorid, Trimethylbenzylammoniumchlorid, Tetraethylammoniumbromid, Tetraethylammoniumchlorid.

Die Reaktionstemperatur kann innerhalb eines größeren Bereiches variiert werden. Sie liegt, wie oben angegeben, zwischen -50°C und +150°C, vorzugsweise zwischen -20°C und +100°C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man pro Mol sulfonyliertem Heteroaromaten der Formel (II) im allgemeinen 0,1 bis 10 Mol, vorzugsweise 0,8 bis 1,2 Mol an Oxyacetamid der Formel (III) und 0,5 bis 10 Mol, vorzugsweise 0,5 bis 5 Mol an Base ein. Die Zugabereihenfolge der Reaktanten kann beliebig vertauscht werden, es können auch alle Komponenten gleichzeitig in das Reaktionsgefäß eindosiert werden. Die Reaktionsführung kann kontinuierlich oder diskontinuierlich gestaltet werden. Die Aufarbeitung erfolgt in üblicher Art und Weise.

Die nach dem erfindungsgemäßen Verfahren herstellbaren Verbindungen der Formel (I) können bekanntermaßen als Herbizide verwendet werden (vgl. z. B. EP-A-0 005 501, EP-A-0 018 497, EP-A-0 029 171, EP-A-0 060 426).

**Herstellungsbeispiele**

**Beispiel 1**

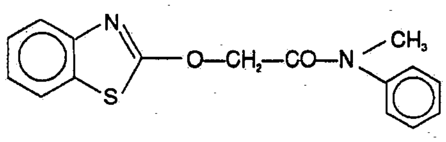

In einem 2-l-Dreihalskolben mit Rührer, Thermometer und Tropftrichter gibt man 227 g (1 Mol) 2-Benzthiazolyl-ethylsulfon und 165 g (1 Mol) Hydroxyessigsäure-N-methyl-anilid zusammen und erwärmt auf 60°C. Die klare Schmelze wird unter Rühren mit 200 g gesättigter Kochsalzlösung verdünnt. Bei 50 - 60°C Innentemperatur tropft man 400 g Natronlauge (45 %ig) zu und läßt danach noch zwei Stunden bei 60°C nachreagieren. Anschließend kühlt man auf Raumtemperatur ab, verdünnt mit 400 ml Wasser und filtriert. Der Rückstand wird auf der Nutsche zweimal mit je 200 ml Wasser gewaschen und anschließend bei 70 - 90°C im Vakuum getrocknet.

Man erhält 283,0 g (95 % der Theorie) 2-(Benzthiazol-2-yloxy)-N-methyl-acetanilid vom Schmelzpunkt 130°C.

Das Ausgangsprodukt 2-Benzthiazolyl-ethylsulfon ist bekannt (vgl. z. B. Chem. Abstr. Vol. *67*, 89998k (1967); Schmelzpunkt 109 - 110°C).

**Beispiel 2**

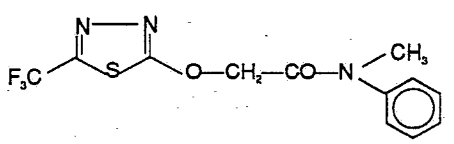

In einem 100-ml-Dreihalskolben mit Rührer und Thermometer werden 5,8 g (0,025 Mol) 2-Methylsulfonyl-5-trifluormethyl-1, 3, 4-thiadiazol, 4,4 g (0,026 Mol) 2-Hydroxyessigsäure-N-methylanilid, 3,8 g (Mol) Kaliumcarbonat, 0,5 g Tetraethylammoniumbromid und 50 ml Aceton vermischt und 20 Stunden bei 20 - 25°C gerührt. Anschließend werden die ungelösten Salze abfiltriert und mit Aceton gewaschen und das gesamte Filtrat wird im Vakuum vom Lösungsmittel befreit. Der Rückstand wird in 100 ml Diethylether aufgenommen, mit verdünnter Salzsäure gewaschen, über Natriumsulfat getrocknet, filtriert und im Vakuum vom Lösungsmittel befreit. Das zurückbleibende Oel kristallisiert nach kurzer Zeit durch. Man erhält 7,2 g (90,8 % der Theorie) 2-(5-Trifluormethyl-1, 3, 4-thiadiazol-2-yloxy-N-methyl-acetanilid vom Schmelzpunkt 63°C.

**Herstellung des Ausgangsproduktes zu Beispiel 2:**

**a) 2-Methylthio-5-trifluormethyl-1, 3, 4-thiadiazol**

In ein Gemisch aus 128 g (1 Mol) Dithiocarbazinsäure-methylester (96 %ig), 260 ml Toluol und 87,1 g (1,1 Mol) Pyridin läßt man bei -5° bis 0°C zuerst 210,7 g (1,53 Mol) Phosphortrichlorid, dann 114 g (1 Mol) Trifluoressigsäure in 90 ml Toluol eintropfen. Das Reaktionsgemisch wird über Nacht bei Raumtemperatur gerührt, dann werden zu dem Gemisch bei Raumtemperatur unter leichter Kühlung 100 ml konz. Schwefelsäure zugetropft und anschließend wird 2 Stunden auf 45 - 55°C erwärmt. Nach dem Abkühlen wird der Ansatz in ein Gemisch aus Eis und Toluol gegossen, die organische Phase wird abgetrennt und die wäßrige Phase wird mit Toluol nachextrahiert. Die toluolische Phase wird im Vakuum eingeengt; es bleiben 169,9 g 2-Methyl-thio-5-strifluormethyl-1, 3, 4-thiadiazol (≘ 82,4 % der Theorie) in Form eines gelblichen Öles mit einem Gehalt von 97 % (gaschromatographisch bestimmt) zurück; Siedepunkt: 44°C/0,67 mbar.

**b) 2-Methylsulfonyl-5-trifluormethyl-1, 3, 4-thiadiazol**

Zu einer Lösung von 20 g (0,097 Mol) 2-Methylthio-5-trifluormethyl-1, 3, 4-thiadiazol (97 %ig) in 70 ml Ameisensäure werden 0,2 g Natriumwolframat zugegeben, anschließend werden bei Raumtemperatur 27 g (0,26 Mol) 30 %iges Wasserstoffperoxid zugetropft; die Temperatur soll dabei 30°C nicht übersteigen. Nach beendeter Zugabe

wird das Reaktionsgemisch 2 Stunden bei 30 - 40°C nachgerührt, dann in Methylenchlorid aufgenommen und nacheinander mit einer wäßrigen Natriumhydrogensulfitlösung und mit Wasser gewaschen. Die organische Phase wird getrocknet und eingeengt. Man erhält 19,1 g 2-Methylsulfonyl-5-trifluormethyl-1, 3, 4-thiadiazol (≅ 85 % der Theorie) in Form weißer Kristalle vom Schmelzpunkt 84°C.

(Vgl. hierzu DE-OS1 817 069 und US-PS3 562 284).

Auf analoge Weise können auch die in den nachfolgenden Tabellen 1, 2a und 2b genannten Verbindungen hergestellt werden:

**Tabelle 1**

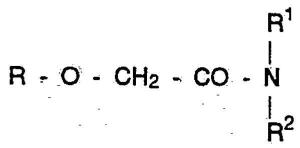

| Beisp. Nr. | R | $R^1$ | $R^2$ | physikalische Daten (Fp. °C / $n_D^{20}$) |
|---|---|---|---|---|
| 3 | 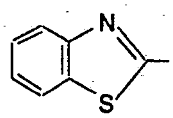 | $-C_2H_5$ | $-C_2H_5$ | 54 |
| 4 | 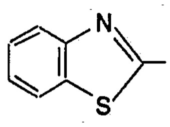 | $-CH_3$ | $-CH_3$ | 129 |
| 5 | 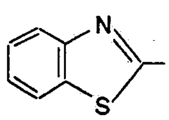 | $-CH_2-CH=CH_2$ | $-CH_2-CH=CH_2$ | 1.5781 |
| 6 | 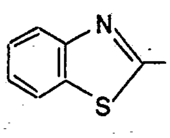 | $-CH_2-CH_2-CH_2-CH_2-CH_2-CH-$<br>$CH_3$ | | 81 |
| 7 | 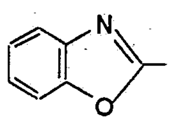 | $C_2H_5$ | 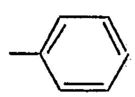 | 125 |
| 8 | 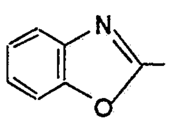 | $CH_3$ | 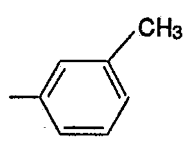 | 118 |
| 9 | 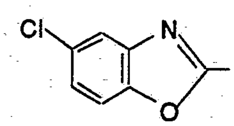 | $-CH_2-CH_2-CH_2-CH_2-CH_2-CH_2-$ | | 98 |
| 10 | 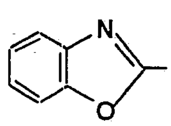 | $CH_3$ | 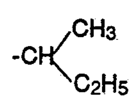 | 1.5983 |
| 11 | 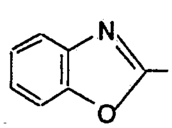 | $CH_3$ | 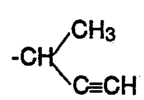 | 93 |

**Tabelle 1**

**Fortsetzung:**

| Beisp. Nr. | R | $R^1$ | $R^2$ | physikalische Daten (Fp. °C / $n_D^{20}$) |
|---|---|---|---|---|
| 12 | 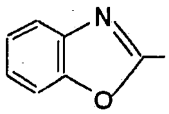 | $-CH_2-CH_2-OCH_3$ | $-CH_2-CH_2-OCH_3$ | Oel |
| 13 | 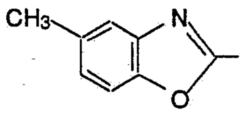 | $CH_3$ | 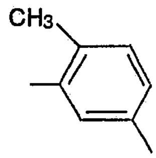 | 166 |
| 14 | 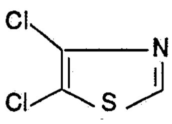 | $CH_3$ | 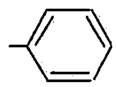 | 88 |
| 15 | 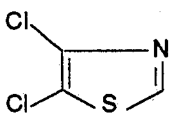 | $CH_3$ | $CH_3$ | 85 |
| 16 | 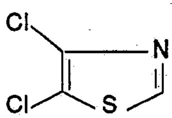 | $C_2H_5$ | $C_2H_5$ | 1.5394 |
| 17 | 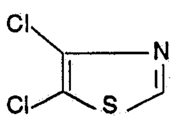 | $CH_3$ | 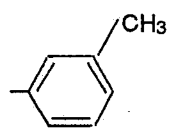 | 75 |
| 18 | 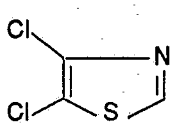 | $-CH_2-CH_2-CH_2-CH_2-CH-$ | | 1.5449 |
| 19 | 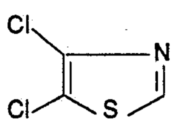 | $-CH_2-CH_2-CH-CH_2-CH_2-$<br>\|<br>$CH_3$ | | 98 |
| 20 | 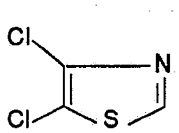 | $-CH_2-CH=CH_2$ | $-CH_2-CH=CH_2$ | 1.5418 |
| 21 | 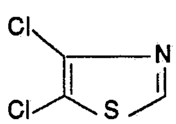 | $CH_3$ | $-CH-C\equiv CH$<br>\|<br>$CH_3$ | Oel |
| 22 | 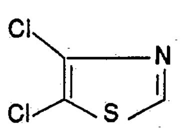 | $CH_3$ | 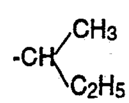 | 1.5357 |

**Tabelle 1**

**Fortsetzung:**

| Beisp. Nr. | R | $R^1$ | $R^2$ | physikalische Daten (Fp. °C / $n_D^{20}$) |
|---|---|---|---|---|
| 23 | 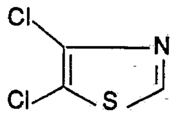 | 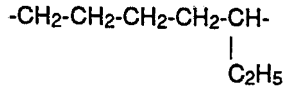 | | 1.5440 |
| 24 | 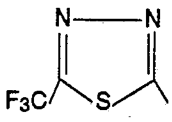 | $CH_3$ | 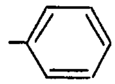 | Oel |
| 25 | 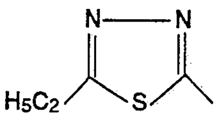 | 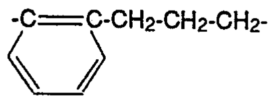 | | 1.5865 |
| 26 | 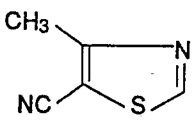 | $CH_3$ | 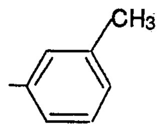 | 87-88 |
| 27 | 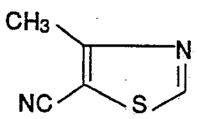 | $CH_3$ | 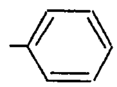 | 92-94 |
| 28 | 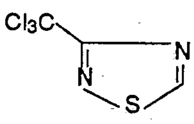 | $OCH_2$ | 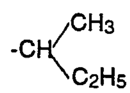 | 1.5230 |
| 29 | 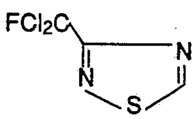 | $CH_3$ | $C_6H_5$ | 67 |
| 30 | 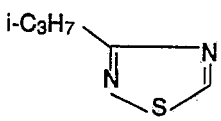 | $CH_3$ | 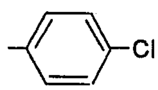 | 86 |
| 31 | 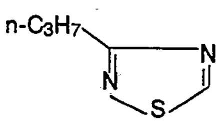 | $CH_3$ | 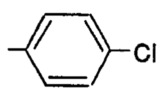 | 55 |
| 32 | 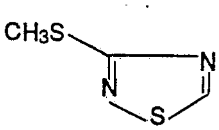 | $CH_3$ | 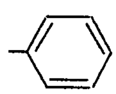 | 98 |
| 33 | 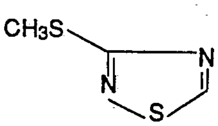 | 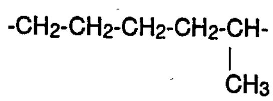 | | 1.5560 |

**Tabelle 1**

**Fortsetzung:**

| Beisp. Nr. | R | $R^1$ | $R^2$ | physikalische Daten (Fp. °C / $n_D^{20}$) |
|---|---|---|---|---|
| 34 | 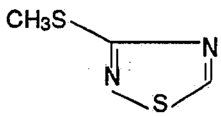 | $-CH_2-CH=CH_2$ | $-CH_2-CH=CH_2$ | 1.5549 |
| 35 | 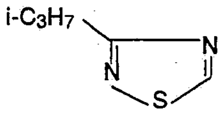 | $-CH_2-CH_2-CH_2-CH_2-CH-$<br>\|<br>$C_2H_5$ | | 67 |
| 36 | 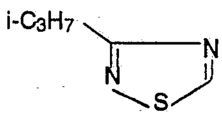 | $-CH_2-CH_2-CH_2-CH_2-CH_2-CH_2-$ | | 60 |
| 37 | 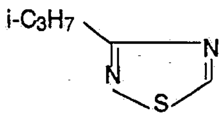 | $CH_3$ | 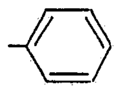 | 47 |
| 38 | 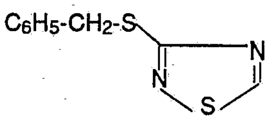 | $-CH_2-CH_2-CH_2-CH_2-CH-$<br>\|<br>$CH_3$ | | 139 |
| 39 | 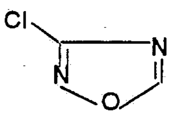 | $-CH_2-CH_2-CH_2-CH_2-CH-$<br>\|<br>$CH_3$ | | 53 |
| 40 | 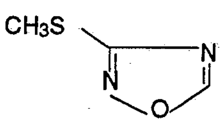 | $CH_3$ | | 192 |
| 41 | 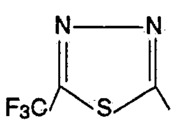 | $-CH_2-CH=CH_2$ | $-CH_2-CH=CH_2$ | |
| 42 | 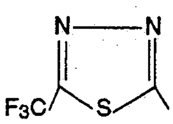 | $-C_3H_7-n$ | $-C_3-H_7-n$ | |

**Tabelle 2a**

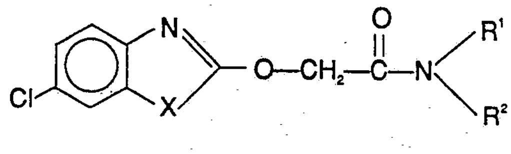

| Bsp. Nr. | $R^1$ | $R^2$ bzw. | 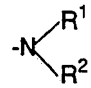 | x | Schmelzpunkt |
|---|---|---|---|---|---|
| 43 | $CH_3$ | $CH_3(CH_2)_3$- | | O | 78-79°C |
| 44 | | | 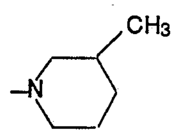 | O | 112°C |
| 45 | | | 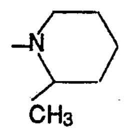 | O | 82°C |
| 46 | | | 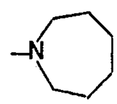 | O | 118°C |
| 47 | $C_2H_5$ | $C_2H_5$ | | O | 80°C |
| 48 | $CH_3$ | $CH_3$ | | O | |
| 49 | $CH_3(CH_2)_2$- | $CH_3(CH_2)_2$- | | O | |
| 50 | $CH_3$ | 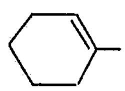 | | O | |
| 51 | $CH_3$ | $CH_3$ | | S | 160°C |
| 52 | $C_2H_5$ | $C_2H_5$ | | S | 76°C |
| 53 | $CH_3$ | $CH_3(CH_2)_3$- | | S | 94°C |
| 54 | $CH_3(CH_2)_2$- | $CH_3(CH_2)_2$- | | S | 71°C |
| 55 | $CH_3$ | 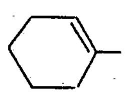 | | S | |
| 56 | | | 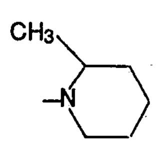 | S | 78°C |
| 57 | | | 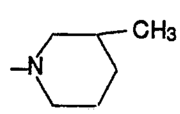 | S | 98°C |
| 58 | | | 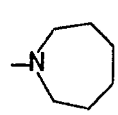 | S | 84°C |
| 59 | $CH_2$=CH-$CH_2$- | $CH_2$=CH-$CH_2$- | | S | 70°C |

**Tabelle 2a**

**Fortsetzung**

| Bsp. Nr. | $R^1$ | $R^2$ | bzw. 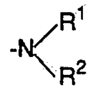 | x | Schmelzpunkt |
|---|---|---|---|---|---|
| 60 | | | 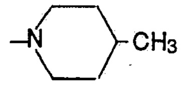 | S | 88°C |
| 61 | $CH_3O$- | 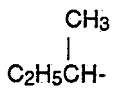 | | S | |
| 62 | $CH_3$ | 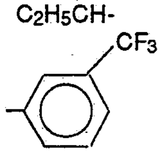 | | S | 144°C |
| 63 | $CH_3$ | $CH\equiv C\text{-}CH_2$ | | S | 115°C |
| 64 | | | 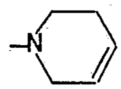 | S | 142°C |
| 65 | | | 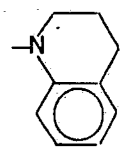 | S | 162°C |
| 66 | $CH_3$ | $CH_3(CH_2)_2$- | | S | |
| 67 | $CH_3$ | $CH_3OCH_2$- | | S | 77°C |
| 68 | $CH_3$ | 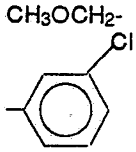 | | S | 123°C |
| 69 | $CH_3$ | 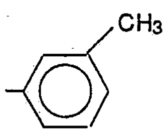 | | S | 126°C |
| 70 | $CH_3(CH_2)_3$- | $CH_3(CH_2)_3$- | | S | 67°C |
| 71 | $C_2H_5$- | $CH_2{=}CH$- | | S | 78°C |
| 72 | $CH_3$ | 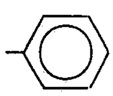 | | S | 108°C |
| 73 | $CH_3$ | 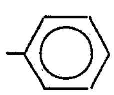 | | O | 139°C |

Tabelle 2b

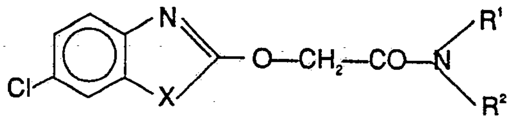

| Bsp. Nr. | X | $R^1$ | $R^2$ | bzw. 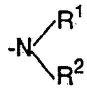 |
|---|---|---|---|---|
| 74 | O | $CH_3$ | $CH_30-CH_2-$ | |
| 75 | O | $CH_3$ | 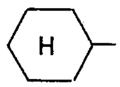 | |
| 76 | O | $CH_3$ | 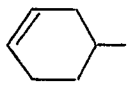 | |
| 77 | O | $CH_3$ | $F_3C-CH_2-$ | |
| 78 | O | $CH_3$ | 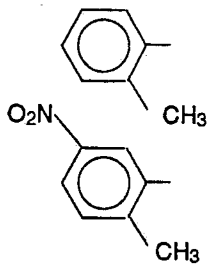 | |
| 79 | O | $CH_3$ | | |
| 80 | O | $CH_3$ | 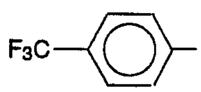 | |
| 81 | O | $C_2H_5$ | $CH_2=CH-CH_2-$ | |
| 82 | O | $CH_2=CH-CH_2-$ | $CH_2=CH-CH_2-$ | |
| 83 | O | | | 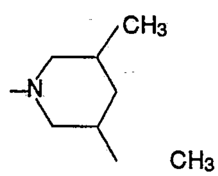 |
| 84 | O | | | 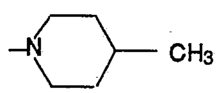 |
| 85 | O | | | 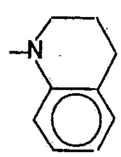 |
| 86 | O | | | 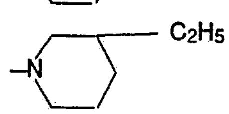 |

**Tabelle 2b**

**Fortsetzung**

| Bsp. Nr. | X | $R^1$ | $R^2$ bzw. 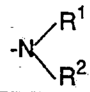 |
|---|---|---|---|
| 87 | O | $CH_3$ | $HC{\equiv}C-CH_2-$ |
| 88 | O | $CH_3O$ | 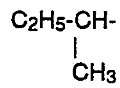 |
| 89 | O | $(CH_3)_2CHO-$ | $C_2H_5O-CH_2CH_2-O-$ |
| 90 | O | $CH_3$ | 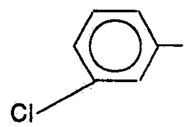 |
| 100 | O | $CH_3$ | 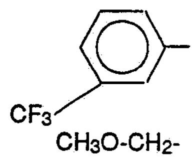 |
| | S | $CH_3$ | $CH_3O-CH_2-$ |
| | S | $CH_3$ | $HC{\equiv}C-CH_2-$ |
| 101 | S | $CH_3$ | 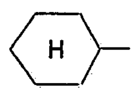 |
| 102 | S | $CH_3$ | 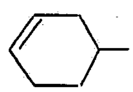 |
| 103 | S | $CH_3$ | $F_3C-CH_2-$ |
| 104 | S | $CH_3$ | 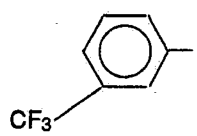 |
| 105 | S | $CH_3$ | 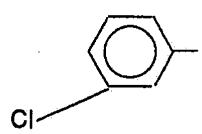 |
| 106 | S | $CH_3$ | 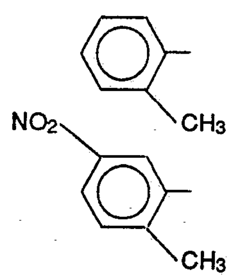 |
| 107 | S | $CH_3$ | |
| 108 | S | $CH_3-$ | 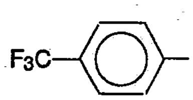 |

**Tabelle 2b**

**Fortsetzung**

| Bsp. Nr. | X | $R^1$ | $R^2$ bzw. 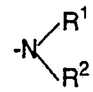 |
|---|---|---|---|
| 109 | S | $CH_3O$- | $C_2H_5$-CH-<br>\|<br>$CH_3$ |
| 110 | S | $(CH_3)_2CHO$- | $(CH_3)_2CH$- |
| 111 | S | $C_2H_5$ | $CH_2$=CH-$CH_2$- |
| 112 | S | | 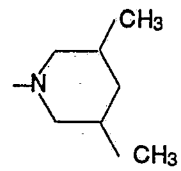 |
| 113 | S | | 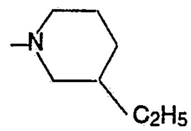 |
| 114 | O | | 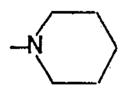 |
| 115 | S | | 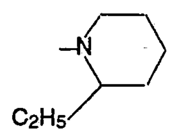 |
| 116 | O | | 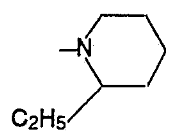 |
| 117 | O | $CH_3$- | $CH_2$=CH-$CH_2$- |
| 118 | S | $CH_3$- | $CH_2$=CH-$CH_2$- |
| 119 | O | $CH_3O$-$CH_2$- | $C_2H_5$-CH-<br>\|<br>$CH_3$ |
| 120 | S | $CH_3O$-$CH_2$- | $C_2H_5$-CH-<br>\|<br>$CH_3$ |
| 121 | O | $CH_3(CH_2)_3$- | $CH_3(CH_2)_3$- |
| 122 | O | $C_2H_5$- | $(CH_3)_2CH$- |
| 123 | S | $C_2H_5$- | $(CH_3)_2CH$- |
| 124 | O | $(CH_3)_2CH$- | $C_2H_5OCH_2CH_2$-O- |
| 125 | S | $(CH_3)_2CH$- | $C_2H_5OCH_2CH_2$-O- |

**Tabelle 2b**

**Fortsetzung**

| Bsp. Nr. | X | $R^1$ | $R^2$ bzw. 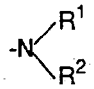 |
|---|---|---|---|
| 126 | O | $CH_3$- | 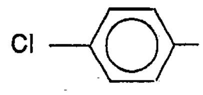 |
| 127 | S | $CH_3$- | 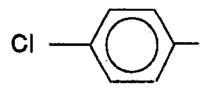 |
| 128 | S | $CH_3$- | 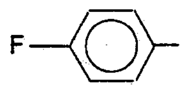 |
| 129 | S | $CH_3$- | 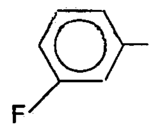 |
| 130 | O | $CH_3$- | 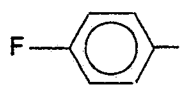 |
| 131 | O | $CH_3$- | 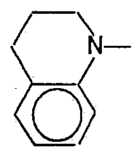 |
| 132 | S | $CH_3$- | 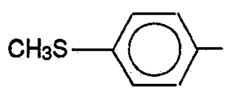 |
| 133 | O | $CH_3$- | 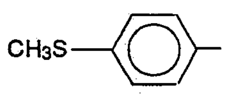 |
| 134 | S | $CH_3$- | 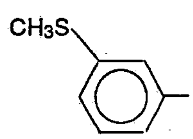 |
| 135 | S | $CH_3$- | 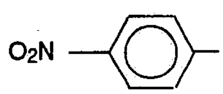 |
| 136 | O | $CH_3$- | 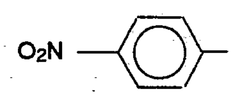 |
| 137 | O | $CH_3$- | n-$H_9C_4$- |
| 138 | S | $CH_3$- | n-$H_9C_4$- |

**Tabelle 2b**

**Fortsetzung**

| Bsp. Nr. | X | $R^1$ | $R^2$ bzw. 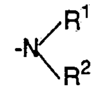 |
|---|---|---|---|
| 139 | O | | 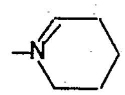 |
| 140 | S | | 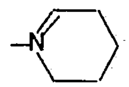 |
| 141 | O | | 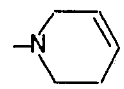 |

## Patentansprüche

1. Verfahren zur Herstellung von Heteroaryloxyacetamiden der allgemeinen Formel (I)

$$R - O - CH_2 - CO - N\begin{matrix} R^1 \\ | \\ \\ | \\ R^2 \end{matrix} \qquad (I)$$

in welcher

R für einen gegebenenfalls einfach oder mehrfach, gleich oder verschieden substituierten 5-gliedrigen aromatischen Heterocyclus steht, der auch benzoannelliert sein kann und der als Heteroatome 1 bis 3 Stickstoffatome und zusätzlich ein Sauerstoffatom oder ein Schwefelatom enthalten kann, wobei als Substituenten infrage kommen: Halogen, Cyano, Nitro, geradkettiges oder verzweigtes Alkyl, Alkoxy oder Alkylthio mit jeweils bis zu 4 Kohlenstoffatomen, geradkettiges oder verzweigtes Halogenalkyl mit bis zu 4 Kohlenstoffatomen und bis zu 9 gleichen oder verschiedenen Halogenatomen, Aralkyl, Aralkoxy oder Aralkylthio mit jeweils 6 oder 10 Kohlenstoffatomen im Arylteil und 1 bis 2 Kohlenstoffatomen im Alkylteil sowie gegebenenfalls ein- bis dreifach, gleich oder verschieden durch $C_{1-4}$-Alkyl, $C_{1-4}$-Alkoxy, Halogen oder $C_{1-4}$-Halogenalkyl substituiertes Aryl mit 6 oder 10 Kohlenstoffatomen, und

$R^1$ und $R^2$ unabhängig voneinander für Wasserstoff, geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatomen (wobei als Substituenten insbesondere Alkylreste mit 1 bis 4 Kohlenstoffatomen in Frage kommen), für geradkettiges oder verzweigtes Alkoxy und Alkoxyalkyl mit 1 bis 8 Kohlenstoffatomen, für Halogenalkyl mit 1 bis 8 Kohlenstoffatomen und 1 bis 5 Halogenatomen (insbesondere Fluor, Chlor und Brom), für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 oder 10 Kohlenstoffatomen stehen, wobei als Substituenten in Frage kommen: Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, insbesondere Fluor, Chlor oder Brom, sowie Nitro, oder

$R^1$ und $R2$ zusammen mit dem Stickstoffatom, an das sie gebunden sind, für einen gegebenenfalls einfach oder mehrfach, gleich oder verschieden substituierten gesättigten oder ungesättigten, 5- bis 7-gliedrigen Heterocyclus stehen, der bis zu 2 weitere Heteroatome, insbesondere Stickstoff oder Sauerstoff enthalten kann, wobei als Substituenten in Frage kommen: geradkettiges oder verzweigtes Alkyl mit 1 bis 6 Kohlenstoffatomen, auch in Form eines annellierten Ringsystems, Aryl mit 6 oder 10 Kohlenstoffatomen, auch in Form eines annellierten Ringsystems oder Dioxyalkylen mit 2 bis 3 Kohlenstoffatomen, dadurch gekennzeichnet, daß man sulfonylierte 5-gliedrige Heteroaromaten der allgemeinen Formel (II),

$$R - SO_2 - R' \qquad (II)$$

in welcher
R die oben angegebene Bedeutung hat und
R' für niederes Alkyl oder für gegebenenfalls substituiertes Benzyl steht,
mit α-Oxyacetamiden der allgemeinen Formel (III),

$$R''-O-CH_2-CO-N(R^1)(R_2) \qquad (III)$$

in welcher
R" für Wasserstoff oder Acyl steht und
$R^1$ und $R^2$ die oben angegebene Bedeutung haben,
in Gegenwart einer anorganischen Base als Säureakzeptor und eines organischen oder anorganischen Lösungsmittels als Verdünnungsmittel, gegebenenfalls in Gegenwart eines Phasentransferkatalysators, bei Temperaturen von -50°C bis +150°C umsetzt.

2. Verfahren nach Anspruch 1 dadurch gekennzeichnet, daß man die Umsetzung bei Temperaturen zwischen -20° und +100°C durchführt.

3. Verfahren nach Anspruch 1 dadurch gekennzeichnet, daß man 2-Benzthiazolyl-methylsulfon oder 2-Benzthiazolyl-ethylsulfon mit Hydroxyessigsäure-N-methylanilid umsetzt.

4. Verfahren nach Anspruch 1 dadurch gekennzeichnet, daß man 2-Methylsulfonyl-5-trifluormethyl-1, 3, 4-thiadiazol oder 2-Ethylsulfonyl-5-trifluormethyl-1, 3, 4-thiadiazol mit Hydroxyessigsäure-N-methylanilid umsetzt.

**Claims**

1. Process for the preparation of heteroaryloxyacetamides of the general formula (I)

$$R-O-CH_2-CO-N(R^1)(R^2) \qquad (I)$$

in which
R represents a 5-membered aromatic heterocycle optionally substituted by one or more identical or different substituents, which can also be benzo-fused and which can contain, as heteroatoms, 1 to 3 nitrogen atoms and in addition an oxygen atom or a sulphur atom, the following being suitable substituents: halogen, cyano, nitro, straight-chain or branched alkyl, alkoxy or alkylthio each having up to 4 carbon atoms, straight-chain or branched halogeno-alkyl having up to 4 carbon atoms and up to 9 identical or different halogen atoms, aralkyl, aralkoxy or aralkylthio each having 6 or 10 carbon atoms in the aryl moiety and 1 to 2 carbon atoms in the alkyl moiety, as well as aryl having 6 or 10 carbon atoms, which is optionally substituted by one to three identical or different substituents chosen from $C_{1-4}$-alkyl, $C_{1-4}$-alkoxy, halogen or $C_{1-4}$-halogenoalkyl, and
$R^1$ and $R^2$ independently of one another represent hydrogen, straight-chain or branched alkyl having 1 to 8 carbon atoms, straight-chain or branched alkenyl and alkynyl each having 2 to 8 carbon atoms, cycloalkyl or cycloalkenyl each having 3 to 7 carbon atoms, which are optionally substituted by one or more identical or different substituents (suitable substituents being, in particular, alkyl radicals having 1 to 4 carbon atoms), straight-chain or branched alkoxy and alkoxyalkyl having 1 to 8 carbon atoms, halogenoalkyl having 1 to 8 carbon atoms and 1 to 5 halogen atoms (in particular fluorine, chlorine and bromine), aralkyl having 6 to 10 carbon atoms in the aryl moiety and 1 to 2 carbon atoms in the alkyl moiety, as well as aryl having 6 or 10 carbon atoms, which is optionally substituted by one or more identical or different substituents, the following being suitable substituents: halogen, straight-chain or branched alkyl, alkoxy or alkylthio each having 1 to 4 carbon atoms, halogenoalkyl, halogenoalkoxy and halogenoalkylthio each having 1 to 2 carbon atoms and 1 to 5 halogen atoms, in particular fluorine, chlorine or bromine, as well as nitro, or
$R^1$ and $R^2$, together with the nitrogen atom to which they are bonded, represent a saturated or unsaturated, 5-membered to 7-membered heterocycle optionally substituted by one or more identical or different substituents, which can contain up to 2 further heteroatoms, in particular nitrogen or oxygen, the following being suitable substituents: straight-chain or branched alkyl having 1 to 6 carbon atoms, also in the form of a fused ring system, aryl having 6 or 10 carbon atoms, also in the form of a fused ring system, or dioxyalkylene having 2 to 3 carbon atoms, characterized in that sulphonylated 5-membered heteroaromatics of the general formula (II)

$R\text{-}SO_2\text{-}R'$ (II)

in which

R has the meaning given above and R' represents lower alkyl or optionally substituted benzyl, are reacted with α-oxyacetamides of the general formula (III)

$$R''\text{-}O\text{-}CH_2\text{-}CO\text{-}N(R^1)(R^2) \qquad (III)$$

in which

R" represents hydrogen or acyl and $R^1$ and $R^2$ have the meanings given above, in the presence of an inorganic base as an acid acceptor and of an organic or inorganic solvent as a diluent, if appropriate in the presence of a phase transfer catalyst, at temperatures of -50°C to +150°C.

2. Process according to claim 1, characterized in that the reaction is carried out at temperatures of between -20° and +100°C.

3. Process according to claim 1, characterized in that benzthiazol-2-yl methyl sulphone or benzthiazol-2-yl ethyl sulphone is reacted with N-methylhydroxyacetanilide.

4. Process according to claim 1, characterized in that 2-methylsulphonyl-5-trifluoromethyl-1, 3, 4-thiadiazole or 2-ethylsulphonyl-5-trifluoromethyl-1, 3, 4-thiadiazole is reacted with N-methylhydroxyacetanilide.

**Revendications**

1. Procédé de production d'hétéroaryloxyacétamides de formule générale (I)

$$R\text{-}O\text{-}CH_2\text{-}CO\text{-}N(R^1)(R^2) \qquad (I)$$

dans laquelle

R représente un hétérocycle aromatique pentagonal portant éventuellement un ou plusieurs substituants identiques ou différents, qui peut aussi être condensé au benzène et qui peut contenir comme hétéroatomes 1 à 3 atomes d'azote et, en outre, 1 atome d'oxygène ou 1 atome de soufre, et on considère alors comme substituants: un halogène, un groupe cyano, nitro, alkyle, alkoxy ou alkylthio à chaîne droite ou ramifiée ayant chacun jusqu'à 4 atomes de carbone, un groupe halogénalkyle à chaîne droite ou ramifiée ayant jusqu'à 4 atomes de carbone et jusqu'à 9 atomes d'halogènes identiques ou différents, un groupe aralkyle, aralkoxy ou aralkylthio ayant chacun 6 ou 10 atomes de carbone dans la partie aryle et 1 ou 2 atomes de carbone dans la partie alkyle, ainsi qu'un groupe aryle de 6 ou 10 atomes de carbone portant éventuellement un à trois substituants alkyle en $C_1$ à $C_4$, alkoxy en $C_1$ à $C_4$, halogéno ou halogénalkyle en $C_1$ à $C_4$ identiques ou différents, et

$R^1$ et $R^2$ représentent, indépendamment l'un de l'autre, l'hydrogène, un groupe alkyle à chaîne droite ou ramifiée ayant 1 à 8 atomes de carbone, un groupe alcényle ou alcynyle à chaîne droite ou ramifiée ayant chacun 2 à 8 atomes de carbone, un groupe cycloalkyle ou cycloalcényle ayant chacun 3 à 7 atomes de carbone, portant éventuellement un ou plusieurs substituants identiques ou différents (on considère alors comme substituants en particulier des restes alkyle de 1 à 4 atomes de carbone), un groupe alkoxy et un groupe alkoxyalkyle à chaîne droite ou ramifiée ayant 1 à 8 atomes de carbone, un groupe halogénalkyle ayant 1 à 8 atomes de carbone et 1 à 5 atomes d'halogènes (notamment fluor, chlore et brome), un groupe aralkyle de 6 à 10 atomes de carbone dans la partie aryle et 1 ou 2 atomes de carbone dans la partie alkyle, ainsi qu'un groupe aryle de 6 ou 10 atomes de carbone portant éventuellement un ou plusieurs substituants identiques ou différents, et on considère alors comme substituants:

un halogène, un groupe alkyle, alkoxy ou alkylthio à chaîne droite ou à chaîne ramifiée ayant chacun 1 à 4 atomes de carbone, un groupe halogénalkyle, halogénalkoxy ou halogénalkylthio ayant chacun 1 ou 2 atomes de carbone et 1 à 5 atomes d'halogènes, notamment fluor, chlore ou brome, ainsi que le groupe nitro, ou bien

$R^1$ et $R^2$ forment conjointement avec l'atome d'azote auquel ils sont liés un hétérocycle pentagonal à heptagonal saturé ou non saturé portant éventuellement un ou plusieurs substituants identiques ou différents, qui peut contenir jusqu'à deux autres hétéroatomes, notamment d'azote ou d'oxygène, et on considère alors comme substituants: un groupe alkyle à chaîne droite ou ramifiée ayant 1 à 6 atomes de carbone, également sous la forme d'un système cyclique condensé, un groupe aryle de 6 ou 10 atomes de carbone, également sous la

forme d'un système cyclique condensé, ou un groupe dioxyalkylène de 2 ou 3 atomes de carbone, caractérisé en ce qu'on fait réagir des composés hétéroaromatiques pentagonaux sulfonylés de formule générale (II)

$$R - SO_2 - R' \qquad (II)$$

dans laquelle

R a la définition indiquée ci-dessus et R' désigne un groupe alkyle inférieur ou un groupe benzyle éventuellement substitué, avec des α-oxyacétamides de formule générale (III)

$$R'' - O - CH_2 - CO - N(R^1)(R^2) \qquad (III)$$

dans laquelle

R" est l'hydrogène ou un groupe acyle et $R^1$ et $R^2$ ont la définition indiquée ci-dessus, en présence d'une base inorganique comme accepteur d'acide et d'un solvant organique ou inorganique comme diluant, le cas échéant en présence d'un catalyseur de transfert de phase, à des températures de -50°C à +150°C.

2. Procédé suivant la revendication 1, caractérisé en ce qu'on conduit la réaction à des températures comprises entre -20 et +100°C.

3. Procédé suivant la revendication 1, caractérisé en ce qu'on fait réagir la 2-benzothiazolyl-méthylsulfone ou la 2-benzothiazolyl-éthylsulfone avec le N-méthylanilide de l'acide hydroxyacétique.

4. Procédé suivant la revendication 1, caractérisé en ce qu'on fait réagir le 2-méthylsulfonyl-5-trifluorométhyl-1, 3, 4-thiadiazole ou le 2-éthylsulfonyl-5-trifluorométhyl-1, 3, 4-thiadiazole avec le N-méthylanilide de l'acide hydroxyacétique.